# EUROPEAN PATENT APPLICATION

(11) **EP 2 641 968 A2**
(43) Date of publication of application: **25.09.2013**
(21) Application number: 11841178.4
(22) Date of filing: 15.11.2011
(51) Int. Cl.: C12N 9/40, A61K 38/43, A61P 1/00

(54) **ORALLY ADMINISTERED PHARMACEUTICAL COMPOSITION AND PREPARATION METHOD THEREOF, FOR THE PREVENTION AND TREATMENT OF IRRITABLE BOWEL SYNDROME, COMPRISING AN INTESTINAL MOTILITY MODIFIER AND -D-GALACTOSIDASE**

(30) Priority: 16.11.2010 MX 2010012480
(71) Applicant: Posi Visionary Solutions LLP, Londres WC1R 4HE (GB)
(72) Inventor: BERNARDO ESCUDERO, Roberto, Hidalgo (MX); SAVOIR VILBOUEF, John Claude, C.P.03100 México D.F. (MX)
(74) Representative: Scott, Susan Margaret
(86) International application number: PCT/MX2011/000139
(87) International publication number: WO 2012/067482

(57) **Abstract**

The invention relates to a pharmaceutical formulation or composition in the form of a tablet, coated tablet, capsule or powder for reconstitution, for use in irritable bowel syndrome, comprising an intestinal motility modifier and enzyme α-D-galactosidase.

## Description

### TECHNICAL FIELD

This invention refers to a pharmaceutical composition or formulation in the form of a tablet, coated tablet, capsule or reconstitutable powder for its use in irritable bowel syndrome, also known as irritable colon syndrome, based on an intestinal motility modifier and the enzyme α-D-galactosidase.

### BACKGROUND

Irritable bowel syndrome (IBS), previously known as irritable colon syndrome, is a functional disorder of the intestine, characterized by symptoms of intestinal discomfort or pain which are associated with alterations of intestinal habits. Currently IBS is understood as being the result of the interaction of many factors that contribute to the appearance of common symptoms, more than an illness with a specific etiology. It is important to note that there is no single physiopathological mechanism that explains the etiology of IBS; however, in patients who suffer from it, nonetheless, there are at least three interrelated factors that present themselves, in ways that vary from individual to individual.

The three factors are:
- Altered intestinal reactivity, its motility or secretion, in response to luminal provocative stimuli (food, distention, inflammation, bacterial factors) or environmental stimuli (psychosocial stress), which result in symptoms of diarrhea or constipation.
- Hypersensitive intestine with increased visceral perception and pain.
- Changes in the regulation of the "brain-intestine" axis.

The diagnosis of IBS is based on the identification of positive symptoms, also known as Roma III Criteria (Longstreth, G.F. 2006. Functional bowel disorders. Gastroenterology. Vol. 130, No. 5:1480-91) and in the ruling out of other illnesses of the intestinal tract with similar symptoms. These criteria are:

Recurring abdominal discomfort or pain of at least three days a month, for the last three months, associated with two or more of the following conditions: a) improvement with defecation, b) onset associated with a change in the frequency of bowel movements, c) onset associated with a change in the appearance of stool.

In which discomfort means an unpleasant sensation not described as pain.

IBS can be considered when presented cases comply with the criteria in the previous three months, with the onset of symptoms at least six months before the diagnosis.

IBS is one of the world's most common health disorders, occurring more frequently in women between 30 and 50 years of age, with a prevalence in Latin America of between 9 and 18% (Schmulson, Max J. 2008. Limited diagnostic close examination can reduce the direct economic impact of irritable bowel syndrome (IBS). Rev Med Chile. Vol. 136: 1398-1405).

The symptomatic pattern in Mexico is of IBS with constipation; abdominal distention is a common symptom in this pattern of the illness. In the Mexican population, abdominal distention and gas are reported as symptoms with very high frequency. Irritable bowel syndrome is a real pathological entity that has a significant impact on the lives of those who suffer from it (severity of symptoms, functional impairment, diminished quality of life), in addition to being a serious economic burden for society and the state, in terms of the costs of medical attention and absenteeism in the workforce (American Gastroenterological Association. 2002. American Gastroenterological Association position statement: irritable bowel syndrome. Gastroenterology.; Vol. 123, No.6:2105-7).

There is no ideal or standard treatment for this illness; however, trimebutine maleate has been used, known commonly as trimebutine, since 1969 for the treatment of functional intestinal disorders, including irritable bowel syndrome. Its principal effects are the regularization of intestinal motility and the elevation of the pain threshold provoked by visceral distension (Roman F.J., et al. 1999. Pharmacological properties of trimebutine and N-monodesmethyltrimebutine. J Pharmacol Exp. Ther.; Vol. 289, No. 3:1391-1397).

Abdominal pain, distention, and flatulence represent very frequent symptoms in functional intestinal disorders, including irritable bowel syndrome, but their physiopathology and treatment have not been fully explained. Patients frequently associate these symptoms with an excessive production of gas in the intestine and the reduction of the latter could represent an effective strategy in the symptomatic improvement of irritable bowel syndrome. It is important to note that these symptoms can emerge or worsen in a patient with irritable bowel syndrome not only because of the increase in the production of gas, but also because of the "normal" presence of gas in the digestive tract combined with an increased visceral sensitivity. There are currently strategies for the treatment of this problem, such as activated carbon, dietary restrictions and probiotics; however, none of them is ideal and the results obtained from these strategies are contradictory. In this context, the fragmentation of nonabsorbable oligosaccharides that are found in legumes, fruits, and vegetables, before they reach the colon (where they will be fermented by bacterial flora and gas will be produced), can represent an attractive alternative. The administration of α-D-galactosidase can achieve this effect (Di Stefano M., et al. 2007. The effect of oral alpha-galactosidase on intestinal gas production and gas-related symptoms. Dig Dis Sci. January, Vol. 52, No. 1:78-83).

There are pharmaceutical products in existence that modify intestinal motility for use in intestinal disorders, such as:

Trimebutine and its salts, Fenoverine, Mebeverine, Dicycloverine, Pinaverium Bromide, Alosetron, Tegaserod, Loperamide, Phloroglucinol, Trimethylphloroglucinol, Butylscopolamine, Pargeverine.

All the products cited above can be used in combination with α-D-galactosidase to prepare a pharmacological formulation for oral administration to treat, alleviate, and mitigate intestinal disorders, such as irritable bowel syndrome.

In another aspect of the same subject, α-D-galactosidase is produced by several microorganisms; the one which is used as a medication is derived from the nontoxic food grade fungus *Aspergillus niger* (http://www.beanoags.com consulted on April 28, 2009).

The combination of trimebutine and its salts, a regulating agent of intestinal motility with analgesic properties and the α-D-galactosidase enzyme that ferments carbohydrates in the colon, reducing the production of gas (and thus, visceral distention), is proposed as an effective treatment in the reduction of the symptoms in patients with irritable bowel syndrome.

The trimebutine acts on the Auerbach's (myenteric) plexus and Meissner's (submucosal) plexus, on the enkephalinergic receptors responsible for the regulation of peristaltic movements. The trimebutine acts on hypermotility as well as hypomotility, depresses or stimulates peristalsis and leads to a normalization of the intestinal tract. Trimebutine also possesses analgesic (modulates visceral sensitivity), antispasmodic and antiemetic properties (Delvaux M. & Wingate D. 1997. Trimebutine: mechanism of action, effects on gastrointestinal function and clinical results. J. Int. Med. Res. Vol. 25, No. 5:225-46).

The α-D-galactosidase enzyme hydrolyzes nonabsorbable oligosaccharides in the intestinal tract avoiding their fermentation by intestinal bacterial flora (process that produces gas); in reducing the production of intestinal gas, it reduces the visceral distension and, as such, symptoms such as distension, abdominal pain and flatulence (http://www.beanogas.com consulted on April 28, 2009). The α-D-galactosidase hydrolyzes three complex carbohydrates: raffinose, stachyose, and verbacose and changes them into monosaccharides: Glucose, galactose, and fructose, and into disaccharide sucrose (the hydrolysis is instant in normal digestion). The α-D-galactosidase enzyme is not normally produced by humans, for which reason the raffinose, stachyose, or verbacose reach the colon intact, where they are fermented by bacterial flora, a chemical reaction which produces hydrogen and methane (gas). The administration of the enzyme with food breaks up these three oligosaccharides prior to their arrival at the colon, preventing the fermentation and production of gas.

The following are food items with a high content of raffinose, stachyose, and verbacose, several of which are very common in the diet of Mexicans (Di Stefano, M., et al. 2007. The effect of oral alpha-galactosidase on intestinal gas production and gas-related symptoms. Dig Dis Sci. Jan, Vol. 52, No. 1:78-83): Cereals: Rice, wheat, corn, oats, barley, muesli (a mix of cereal with dried fruit).

Legumes: Beans, chickpeas, lentils, peas, soy, broad beans.

Fruits: oranges, bananas, kiwis, grapefruit.

Nuts: peanuts, almonds, hazelnuts, walnuts, pine nuts, pistachios.

Vegetables: asparagus, broccoli, carrots, cabbage, cucumbers, onions, mushrooms, potatoes, peppers, leeks.

Several state-of-the-art solutions have been found for somatic pain and abdominal inflammation, for example, the paper WO2001/047515 reports the use of trimebutine alone to prepare a medication; however, it only focuses on the relief of symptoms of this ailment and not on the ailment itself.

The application MXPA02006376 reports the use of trimebutine alone to prevent or treat somatic pain and inflammation associated with gastric ailments; however, it is limited to the treatment of the symptoms, not to the ailment or the causes that provoke it.

The paper US 2003/0119903 reports the use of trimebutine alone to prepare a medication to prevent or treat inflammatory somatic pain as well as chronic pain, associated with gastric ailments, and once again it does not treat the source of the pain nor the source of inflammation associated with gastric ailments.

The paper US 2004/0009234 reports on a pharmaceutical composition and the associated treatment for preventing gastrointestinal disorders making use of only trimebutine without achieving the prevention of gastrointestinal disorders, since it does not combat the source of said ailments.

The paper MX00PA05010821A reports the use of trimebutine for the treatment of constipation, without achieving the desired final result, as it does not combat the source of these ailments.

The paper W0001995001803 reports the use of trimebutine for the prevention or the treatment of gastrointestinal pain and disorders such as indigestion, excessive ingestion of food, esophageal reflux, dyspepsia and constipation; however, it does not prevent gastrointestinal pain since it does not resolve its causes.

One object of this invention is to provide a pharmaceutical formulation for oral administration, with application in intestinal disorders, based on an intestinal motility modifier and α-D-galactosidase.

Another object of this invention is to provide a pharmaceutical formulation for oral administration, with application in intestinal disorders, based on an intestinal motility modifier and α-D-galactosidase to normalize intestinal passage.

Yet another object of this invention is to provide a pharmaceutical formulation for oral administration, with application in intestinal disorders, based on an intestinal motility modifier and α-D-galactosidase, which is effective in achieving analgesic activity in the treatment of gastrointestinal ailments.

Another object of this invention is to provide a pharmaceutical formulation for oral administration, with application in intestinal disorders, based on an intestinal motility modifier and α-D-galactosidase, which is effective in achieving antispasmodic activity.

An additional object of this invention is to provide a pharmaceutical formulation for oral administration, with application in intestinal disorders, based on an intestinal motility modifier and α-D-galactosidase, which is effective in reducing symptoms related to intestinal gas, such as distension, pain, and flatulence.

### DETAILED DESCRIPTION OF THE INVENTION

The pharmaceutical formulation in the form of a tablet, coated tablet, capsule, or reconstitutable powder to treat irritable bowel syndrome, also known as irritable colon syndrome, based on an intestinal motility modifier and the enzyme α-D-galactosidase, is prepared according to the following procedure:
1. The intestinal motility modifier (100-200 mg) is mixed with the α-D-galactosidase enzyme, a binding agent (2-10%), a diluting agent (20-50%), a disintegrant (1-10%), a lubricant (0.25-5%), and a glidant (0.2-5%).
2. A binder solution is prepared.
3. The intestinal motility modifier, the α-D-galactosidase enzyme, a binding agent, a diluting agent, a disintegrant, a lubricant, and a glidant are sieved through a sieve with a 450 to 2,000 micron mesh in order to break up clumps.
4. All elements mentioned in the prior step are mixed and then moistened with the binder solution.
5. The product resulting from the prior step is ground and dried, and then sieved.
6. If the final composition is solid, the mixture is compressed to form a tablet or a coated tablet; otherwise (if granulated), capsules are prepared.
7. The tablets/capsules are packaged in packing material.

To carry out the specified manufacturing process, one will use the equipment that is conventionally used in the production of a pharmaceutical formulation with the indicated characteristics. All raw materials used are pharmaceutical grade. Some practical examples of how the formulations were prepared are described below:

### EXAMPLES

### Example 1:

| Component | Amount |
|---|---|
| Trimebutine Maleate | 200,000 mg |
| α-D-galactosidase | 450,000 GalU* |
| Hydroxypropyl cellulose | 29,000 mg |
| Lactose hydrous | 18,000 mg |
| Sodium starch glycolate | 19,500 mg |
| Microcrystalline cellulose | 100,000 mg |
| Lactose-cellulose 75:25 | 121,300 mg |
| Talc | 12,000 mg |
| Colloidal silicon dioxide | 2,400 mg |
| Magnesium stearate | 7,800 mg |

| | |
|---|---|
| *450 mg of α-D-galactosidase is equivalent to 450 GalU. GalU refers to the enzymatic activity of the α-D-galactosidase, starting from a raw material with 10,000 GalU per gram. | |

Procedure for Example 1: Production of trimebutine maleate, in combination with α-D-galactosidase, by wet granulation. Intragranular addition of the enzyme,
1. Prepare a binder solution by dispersing 50% of the hydroxypropyl cellulose in a sufficient amount of water.
2. Pass the following raw materials through a sieve with a mesh size of 420 to 2,000 microns:
   - The rest of the hydroxypropyl cellulose (50%)
   - The α-D-galactosidase
   - Trimebutine maleate
   - Lactose hydrous
   - Sodium starch glycolate
   - Microcrystalline cellulose
3. Add the products from the previous step into the mixer/granulator equipment and mix for between 5 and 30 minutes at 50 to 200 rpm.
4. Begin moistening with the binder solution from step 1.
5. Put the product obtained in step 4 through the grinder via sieve with a mesh size of 3,000 to 5,000 microns.
6. Dry the product at a temperature of 30 to 60°C until it reaches a residual humidity of 1.0-3.0%.
7. Grind the product obtained in step 6 using a grinder with a 0.033 to 0.094 inch sieve at a speed of 500 to 1,500 rpm.
8. Mix an amount of Lactose-Cellulose 75:25 approximately equal to that of the colloidal silicon dioxide and mix until a homogeneous distribution is obtained.
9. Pass the colloidal silicon dioxide-lactose-cellulose 75:25 mixture obtained in step 8, the talc, the rest of the lactose-cellulose 75:25, and the magnesium stearate through a sieve with a mesh size of 420 to 2,000 microns.
10. Add the following products to a mixer:

The granules obtained in step 7.

The lactose-cellulose 75:25 obtained in step 9.

The colloidal silicon dioxide-lactose-cellulose 75:25 mixture obtained in step 9.

The talc obtained in step 9.

Mix all of this for 10 to 30 minutes at 15 to 30 rpm.
11. Add the magnesium stearate obtained in step 9 to the mixer and mix it with the product for 5 to 10 minutes at 15 to 30 rpm.
12. Compress the product.

### Example 2:

| Component | Amount |
|---|---|
| Trimebutine Maleate | 200,000 mg |
| α-D-galactosidase | 450,000 GalU* |
| Hydroxypropyl cellulose | 29,000 mg |
| Lactose hydrous | 108,000 mg |
| Sodium starch glycolate | 19,500 mg |
| Microcrystalline cellulose | 100,000 mg |
| Lactose-cellulose 75:25 | 121,300 mg |
| Talc | 12,000 mg |
| Colloidal silicon dioxide | 2,400 mg |
| Magnesium stearate | 7,800 mg |

| | |
|---|---|
| *450 mg of α-D-galactosidase is equivalent to 450 GalU. GalU refers to the enzymatic activity of the α-D-galactosidase, starting from a raw material with 1,000 GalU per gram. | |

Procedure for Example 2: Production of trimebutine maleate, in combination with α-D-galactosidase, by wet granulation. Extragranular addition of the enzyme,
1. Prepare a binder solution by dispersing 50% of the hydroxypropyl cellulose in a sufficient amount of water.
2. Pass the following raw materials through a sieve with a mesh size of 420 to 2,000 microns:
   - The rest of the hydroxypropyl cellulose (50%)
   - Trimebutine maleate
   - Lactose hydrous
   - Sodium starch glycolate
   - Microcrystalline cellulose
3. Add the products from the previous step into the granulator equipment and mix for between 5 and 20 minutes at 50 to 200 rpm.
4. Begin moistening with the binder solution from step 1.
5. Put the product obtained in step 4 through the grinder with a sieve with a mesh size of 3,000 to 5,000 microns.
6. Dry the product at a temperature of 30 to 60°C until it reaches a residual humidity of 1.0-3.0%.
7. Grind the product obtained in step 6 using a grinder with a 0.033 to 0.094 inch sieve and at a speed of 500 to 1,500 rpm.
8. Mix an amount of Lactose-Cellulose 75:25 approximately equal to that of the colloidal silicon dioxide and mix until a homogeneous distribution is obtained.
9. Pass the colloidal silicon dioxide-lactose-cellulose 75:25 mixture obtained in step 8, the talc, the rest of the lactose-cellulose 75:25, and the magnesium stearate through a sieve with a mesh size of 420 to 2,000 microns.
10. Add the following products to a mixer:

The granules obtained in step 7.

The lactose-cellulose 75:25 obtained in step 9.

The α-D-galactosidase.

The colloidal silicon dioxide-lactose-cellulose 75:25 mixture obtained in step 9.

The talc obtained in step 9.

All of this is mixed for 10 to 30 minutes at 15 to 30 rpm.
11. Add the magnesium stearate obtained in step 9 to the mixer and mix it with the product for 5 to 10 minutes at 15 to 30 rpm.
12. Compress the product.

### Example 3:

| Component | Amount |
|---|---|
| Trimebutine Maleate | 200,000 mg |
| α-D-galactosidase | 450,000 GalU* |
| Microcrystalline cellulose | 277,000 mg |
| Croscarmellose sodium | 18,000 mg |
| Talc | 5,400 mg |
| Colloidal silicon dioxide | 1,800 mg |
| Magnesium stearate | 7,800 mg |

| | |
|---|---|
| *90 mg is equivalent to 450 GalU. GalU refers to the enzymatic activity of the α-D-galactosidase, starting from a raw material with 5,000 GalU per gram. | |

Procedure for Example 3: Production of trimebutine maleate, in combination with α-D-galactosidase, by direct compression.
1. Mix an amount of microcrystalline cellulose approximately equal to that of the colloidal silicon dioxide and mix until a homogeneous distribution is obtained.
2. Sieve the mixture of colloidal silicon dioxide-microcrystalline cellulose obtained in step 1 through a sieve with a mesh size of 420 to 2,000 microns.
3. Pass the following raw materials through a sieve with a mesh size of 420 to 2,000 microns:
   - Trimebutine maleate
   - α-D-galactosidase
   - Microcrystalline cellulose
   - Croscarmellose sodium
   - Talc
   - Magnesium stearate
4. Add into a mixer in the following order to ensure a good mix and thus a good homogeneity of the product:
   - 1/3 of the microcrystalline cellulose
   - Half of the α-D-galactosidase
   - Half of the trimebutine maleate
   - Half of the α-D-galactosidase
   - Half of the trimebutine maleate
   - 1/3 of the microcrystalline cellulose
   - The talc
   - The croscarmellose sodium
   - The colloidal silicon dioxide-microcrystalline cellulose mixture
   - The rest of the microcrystalline cellulose
   Mix for 10 to 30 minutes at 15 to 30 rpm.
5. Add the magnesium stearate obtained in step 3 to the mixer and mix with the product for 5 to 10 minutes at 15 to 30 rpm.
6. Compress the product.

### Example 4

| Component | Amount |
|---|---|
| Trimebutine Maleate | 200,000 mg |
| α-D-galactosidase | 450,000 GalU* |
| Microcrystalline cellulose and Sodium carboxymethyl cellulose | 6,78 mg [sic] |
| Sucrose | 400,000 mg |
| Colloidal silicon dioxide | 1,800 mg |
| Magnesium stearate | 7,800 mg |

| | |
|---|---|
| *90 mg is equivalent to 450 GalU. GalU refers to the enzymatic activity of the α-D-galactosidase, starting from a raw material with 10,000 GalU per gram. | |

Procedure for Example 4: Production of trimebutine maleate, in combination with α-D-galactosidase, by powder mixing. Reconstitutable powder.
1. Mix an amount of microcrystalline cellulose approximately equal to that of the colloidal silicon dioxide and mix until a homogeneous distribution is obtained.
2. Sieve the mixture of colloidal silicon dioxide-microcrystalline cellulose obtained in step 1 through a sieve with a mesh size of 420 to 2,000 microns.
3. Pass the following raw materials through a sieve with a mesh size of 420 to 2,000 microns:
   - Trimebutine maleate
   - α-D-galactosidase
   - Microcrystalline cellulose and Sodium carboxymethyl cellulose
   - Sucrose
   - Magnesium stearate
4. Mix until a homogeneous distribution is obtained.
5. Fill the product.

The excipients that can properly fulfill the indicated functions are listed below:

| Function | Excipient |
|---|---|
| Binding agent | Hydroxypropyl cellulose, corn starch, propyl cellulose, methyl cellulose |
| Diluting agent | Lactose, microcrystalline cellulose, dibasic calcium phosphate, mannitol, sucrose |
| Disintegrant | Croscarmellose sodium, corn starch, crospovidone |
| Lubricant | Magnesium stearate, talc, stearic acid |
| Glidant | Colloidal silicon dioxide |
| Suspending agent | Microcrystalline cellulose Sodium carboxymethylcellulose |

- The binding agent is selected from those excipients that provide cohesiveness to the materials in powder form, forming granules.
- The diluting agent is selected from those excipients that have the function of increasing the apparent volume of the powder and, as such, increase the weight of the tablet or capsule.
- The disintegrant is selected from those excipients that are able to break up (disintegrate) the tablet and the granules when they come in contact with liquid.
- The lubricant is selected from those excipients that are able to reduce the friction between the granules and the die wall during the process of compression or filling of capsules.
- The glidant is selected from those excipients that are able to create a flow of the granules from the hopper to the die chamber by reducing the interparticle friction.
- The suspending agent is selected from the excipients that increase viscosity and slow sedimentation.

## Claims

1. A pharmaceutical composition adapted to be administered orally in the form of a tablet, coated tablet, capsule or reconstitutable powder, with application in intestinal disorders based on an intestinal motility modifier and the enzyme α-D-galactosidase, the formulation consists primarily of an intestinal motility modifier and the α-D-galactosidase enzyme, a binding agent, a diluting agent, a lubricant, a glidant, and a disintegrant or a suspending agent.

2. The pharmaceutical composition in accordance with claim 1, wherein the intestinal motility modifier can be trimebutine, fenoverine, mebeverine, dicycloverine, pinaverium bromide, alosetron, tegaserod, loperamide, phloroglucinol, trimethylphloroglucinol, butylscopolamine, pargeverine.

3. The pharmaceutical composition in accordance with claim 2, wherein the intestinal motility modifier can be trimebutine and its acceptable pharmaceutical salts.

4. The pharmaceutical composition in accordance with claim 2, wherein the intestinal motility modifier can be fenoverine and its acceptable pharmaceutical salts.

5. The pharmaceutical composition in accordance with claim 2, wherein the intestinal motility modifier can be mebeverine and its acceptable pharmaceutical salts.

6. The pharmaceutical composition in accordance with claim 2, wherein the intestinal motility modifier can be dicycloverine and its acceptable pharmaceutical salts.

7. The pharmaceutical composition in accordance with claim 2, wherein the intestinal motility modifier can be pinaverium bromide and its acceptable pharmaceutical salts.

8. The pharmaceutical composition in accordance with claim 2, wherein the intestinal motility modifier can be alosetron and its acceptable pharmaceutical salts.

9. The pharmaceutical composition in accordance with claim 2, wherein the intestinal motility modifier can be tegaserod and its acceptable pharmaceutical salts.

10. The pharmaceutical composition in accordance with claim 2, wherein the intestinal motility modifier can be loperamide and its acceptable pharmaceutical salts.

11. The pharmaceutical composition in accordance with claim 2, wherein the intestinal motility modifier can be phloroglucinol.

12. The pharmaceutical composition in accordance with claim 2, wherein the intestinal motility modifier can be trimethylphloroglucinol and its acceptable pharmaceutical salts.

13. The pharmaceutical composition in accordance with claim 2, wherein the intestinal motility modifier can be butylscopolamine and its acceptable pharmaceutical salts.

14. The pharmaceutical composition in accordance with claim 2, wherein the intestinal motility modifier can be pargeverine and its acceptable pharmaceutical salts.

15. The pharmaceutical composition in accordance with claim 1, wherein the enzymatic activity of the α-D-galactosidase is 450 GalU.

16. The pharmaceutical composition in accordance with claim 1, wherein the binding agent is selected from the group consisting of hydroxypropyl cellulose, corn starch, propyl cellulose, and methyl cellulose.

17. The pharmaceutical composition in accordance with clause 1, wherein the diluting agent is selected from the group consisting of lactose, microcrystalline cellulose, dibasic calcium phosphate, mannitol.

18. The pharmaceutical composition in accordance with claim 1, wherein the disintegrants can be croscarmellose sodium, cornstarch, crospovidone.

19. The pharmaceutical composition in accordance with claim 1, wherein the lubricant is selected from the group consisting of magnesium stearate, talc, stearic acid.

20. The pharmaceutical composition in accordance with claim 1, wherein the glidant is colloidal silicon dioxide.

21. The pharmaceutical composition in accordance with claim 1, wherein the suspending agent is microcrystalline cellulose and sodium carboxymethylcellulose.

22. Process for preparing a pharmaceutical composition adapted for oral administration in the form of a reconstitutable powder wherein an intestinal motility modifier, an α-D-galactosidase enzyme, a diluting agent, a lubricant, a glidant, and a suspending agent are sieved.

23. Process for preparing a pharmaceutical composition in accordance with claim 22 wherein the sieving is performed with a sieve with a 420 to 2,000 micron mesh.

24. Process for preparing a pharmaceutical composition in accordance with claim 22 wherein the components are mixed until a homogeneous distribution is obtained.

25. Process for preparing a pharmaceutical composition adapted for oral administration in the form of a tablet, coated tablet, reconstitutable powder or capsule wherein an intestinal motility modifier, an α-D-galactosidase enzyme, a binding agent, a diluting agent, a disintegrant, a lubricant, and a glidant are mixed and then sieved.

26. Process for preparing a pharmaceutical composition in accordance with claim 25 wherein the sieving is performed with a sieve with a 420 to 2,000 micron mesh.

27. Process for preparing a pharmaceutical composition in accordance with claim 25 wherein the components are mixed until a homogeneous distribution is obtained.

28. Process for preparing a pharmaceutical composition adapted for oral administration in the form of a tablet, coated tablet or capsule wherein an intestinal motility modifier, an α-D-galactosidase enzyme, a binding agent, a diluting agent, a disintegrant, a lubricant, and a glidant are mixed and then sieved; the intestinal motility modifier and the α-D-galactosidase are moistened with a previously prepared binder solution; the mixture is then ground, dried, and sieved.

29. Process for preparing a pharmaceutical composition in accordance with clause 28, wherein the intestinal motility modifier can be trimebutine and its acceptable pharmaceutical salts.

30. Process for preparing a pharmaceutical composition in accordance with claim 28, wherein the moistening is performed with a binder solution.

31. Process for preparing a pharmaceutical composition in accordance with claim 30, wherein the binder solution is prepared from a binding agent and water.

32. Process for preparing a pharmaceutical composition in accordance with claim 28, wherein the components of the formulation are mixed until a homogeneous distribution is obtained.

33. Process for preparing a pharmaceutical composition in accordance with claim 28, wherein the intestinal motility modifier and the α-D-galactosidase are moistened with the binder solution.

34. Process for preparing a pharmaceutical composition in accordance with claim 28, wherein the components are dried when they have previously been moistened.

35. Process for preparing a pharmaceutical composition in accordance with claim 34, wherein the components are dried at a temperature of 30 to 60°C.

36. Process for preparing a pharmaceutical composition in accordance with claim 35, wherein the final composition retains a final residual humidity of no more than 5%.

37. Process for preparing a pharmaceutical composition in accordance with claim 36, wherein the product is ground when it has previously been granulated.

38. Process for preparing a pharmaceutical composition in accordance with claim 37, wherein the product is ground with a 420 to 2,000 micron mesh.

39. Process for preparing a pharmaceutical composition in accordance with claim 38, wherein the product is sieved.

40. Process for preparing a pharmaceutical composition in accordance with claim 39, wherein the sieving is performed with a sieve with a 420 to 2,000 micron mesh.

41. Process for preparing a pharmaceutical composition in accordance with claim 40, wherein the lubricant is mixed with the intestinal motility modifier and the α-D-galactosidase.

42. Process for preparing a pharmaceutical composition in accordance with claim 41, wherein the lubricant is mixed with the intestinal motility modifier and the α-D-galactosidase until a homogeneous mixture is obtained.

43. Use of a composition or a pharmaceutical composition in accordance with the preceding claims, adapted for oral administration in the prevention or treatment of intestinal disorders.

44. Use of a composition or a pharmaceutical composition in accordance with the preceding claims, adapted for oral administration in the prevention or treatment of irritable bowel syndrome.

45. Use of a composition or a pharmaceutical composition in accordance with the preceding claims, adapted for oral administration in the prevention or treatment of intestinal disorders to achieve antispasmodic and antiemetic activity.

46. Use of a pharmaceutical composition adapted for oral administration with application in intestinal disorders based on an intestinal motility modifier, a binding agent, a diluting agent, a lubricant, a glidant and a disintegrant or a suspending agent.
